# EUROPEAN PATENT SPECIFICATION

(11) **EP 1 988 902 B1**
(45) Date of publication and mention of the grant of the patent: **23.11.2011**
(21) Application number: 07717010.8
(22) Date of filing: 23.01.2007
(51) Int. Cl.: C07D 295/185, A61K 31/495, A61P 25/28, A61P 25/24, A61P 25/18, A61P 25/22, C07D 295/088

(54) **PIPERAZINE DERIVATIVES**
PIPERAZIN-DERIVATE
DERIVES DE PIPERAZINE

(30) Priority: 15.02.2006 US 773391 P
(43) Date of publication of application: 12.11.2008
(73) Proprietor: M's Science Corporation, Chuo-ku Kobe 650-0047 (JP)
(72) Inventor: SUN, Connie L., Palo Alto, CA 94303 (US); LI, Xiaoyuan, Los Altos, CA 94024 (US)
(74) Representative: Goodfellow, Hugh Robin
(86) International application number: PCT/US2007/002043
(87) International publication number: WO 2007/097871

(56) References cited:
- FR-A- 2 073 277
- US-A- 5 281 598
- US-A- 5 736 546
- KATZ ET AL.: 'Behavioral effects of rimcazole analogues alone and in combination with cocaine' EUROPEAN JOURNAL OF PHARMACOLOGY vol. 468, no. 2, May 2003, pages 109 - 119, XP008129270
- KAWAMURA ET AL.: 'Synthesis and evaluation of 11C- and 18F-labeled 1-[2-(4-alkoxy-3-methoxyphenyl)ethyl]-4-(3- phenylpropyl)piperazine as sigma receptor ligand for positron emission tomography studies' NUCLEAR MEDICINE AND BIOLOGY vol. 30, no. 3, April 2003, pages 273 - 284, XP004425101

## Description

The present invention relates to novel 1,4-piperazine derivatives, to processes for preparing the novel derivatives, to pharmaceutical compositions comprising the derivatives, and to the use of derivatives in the treatment of disorders of the central nervous system.

It has been disclosed in the scientific literature that certain disorders of the central nervous system may be treated using a modulator of sigma receptor function. Amongst compounds known to possess affinity for sigma ligands are certain piperazine derivatives.

WO 91/09594 discloses compounds having affinity for sigma receptors, certain of which are piperazine derivatives, and discloses that they are useful in the treatment of schizophrenia and other psychoses.

United States patent number 5,736,546 discloses certain 1,4-(diphenylalkyl)-piperazines having one phenyl group unsubstituted and the other phenyl group substituted by two alkoxy groups. One of the compounds disclosed is 1-[2-(3,4-dimethoxyphenyl)ethyl)-4-(3-phenylpropyl)piperazine. It is also referred to in the scientific literature as SA 4503. The compounds of US 5,736,546 are said to be useful in the treatment of dementia, depression, schizophrenia, anxiety neurosis, diseases accompanying abnormal immune response, cryptorrhea and digestive ulcer.

WO 2004/110387 discloses that sigma ligands, in particular SA 4503, are also useful in the treatment of patients to facilitate neuronal regeneration after onset of a neurodegenerative disease, such as ischemic stroke, traumatic brain injury or spinal chord injury.

United States patent number 5,389,630 discloses certain diamine compounds having cerebral protective action. The compound of Example 50 is a piperazine derivative, but the vast majority of the exemplified compounds are homopiperazine derivatives. The mechanism of action of the compounds is not discussed.

French patent application publication number FR 2073277 generically discloses certain 1,4-disubstituted piperazine derivatives, which are said to possess a wide variety of pharmacological activities. One of the exemplified compounds is 1,4-bis-[2-(4-fluorophenoxy)ethyl]piperazine.

It has now been found that certain 1,4-piperazine derivatives, including the known compound 1,4-bis-[2-(4-fluorophenoxy)ethyl]piperazine, have high affinity for sigma receptors, in particular sigma-1 receptors.

According to one aspect, the present invention provides a compound of general formula (I) 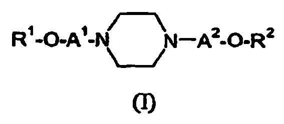 in which:-
R¹ represents a phenyl group that is substituted by one or two fluorine atoms;
A¹ represents (CH₂)ₘ or (CH₂)ₘ₋₁C(=O);
m is 2, 3, 4 or 5;
A² represents (CH₂)ₙ or C(=O)(CH₂)ₙ₋₁;
n is 2, 3, 4 or 5; and
R² represents a phenyl group that is substituted by one, two or three substituents selected independently from (1-2C)alkylenedioxy, a hydroxyl group, a cyano group, a (3-6C)cycloalkyl group, a halo(1-4C) alkyl group, a (1-4C)alkoxy group, a (1-4C)alkoxy(1-4C)alkoxy group, a hydroxy(1-4C)alkoxy group, a (1-4C)alkylsulfanyl group, a (1-4C)alkylsulfonyl group, a halo(1-4C)alkoxy group and a halo(1-4C)alkysulfonyl group;
or a pharmaceutically acceptable salt thereof.

Compounds according to the invention have been found to have high affinity for sigma receptors, in particular sigma-1 receptors.

As used herein, unless otherwise indicated, the term halogen atom includes fluorine, chlorine and bromine.

The term (1-2C)alkylenedioxy includes methylenedioxy and ethylenedioxy.

An example of a (1-4C) alkyl group is methyl.

The term halo(1-4C)alkyl as used herein includes perfluoro(1-4C)alkyl, such as trifluoromethyl.

An example of a (1-4C)alkoxy group is methoxy.

An example of a (1-4C)alkoxy(1-4C)alkoxy group is 2-methoxyethoxy.

An example of a hydroxy(1-4C)alkoxy group is hydroxymethyl.

The term halo(1-4C)alkoxy as used herein includes perfluoro(1-4C)alkoxy, such as trifluoromethoxy.

An example of a (1-4C)alkylsulfanyl group (also known as a (1-4C)alkylthio group) is methylsulfanyl.

An example of a (1-4C)alkylsulfonyl group is methylsulfonyl.

An example of a halo(1-4C)alkylsulfonyl group is trifluoromethylsulfonyl.

Referring to formula (I), examples of particular values for R¹ are 2-fluorophenyl, 3-fluorophenyl, 4-fluorophenyl and 2,4-difluorophenyl.

Examples of values for m are 2 and 3. An example of a particular value for m is 2.

Examples of particular values for n are 2 and 3.

In one embodiment, A¹ is (CH₂)ₘ, such as (CH₂)₂.

In another embodiment, A¹ is (CH₂)ₘ₋₁C(=O), such as (CH₂)C(=O) or (CH₂)₂C(=O).

A² may represent, for example, (CH₂)ₙ.

In a further embodiment, R² represents a phenyl group that is substituted by one or two substituents selected independently from a cyano group, a fluoro(1-4C) alkyl group, a (1-4C)alkylsulfonyl group, a fluoro(1-4C)alkoxy group and a fluoro(1-4C)alkylsulfonyl group.

Examples of particular values for R² are 3-cyanophenyl, 4-cyanophenyl, 3-trifluoromethylphenyl, 4-trifluoromethylphenyl, 3-methylsulfonylphenyl, 4-methylsulfonylphenyl, 3-trifluoromethylsulfonylphenyl and 4-trifluoromethylsulfonylphenyl.

It will be appreciated that certain compounds of formula (I) contain a centre of asymmetry. These compounds may therefore exist and be isolated in the form of stereoisomers. The present invention provides a compound of formula (I) in any stereoisomeric form.

It will also be appreciated that the compounds of formula (I) or their pharmaceutically acceptable salts may be isolated in the form of a solvate, and accordingly that any such solvate is included within the scope of the present invention.

The compounds of general formula (I) can be prepared by conventional processes.

According to another aspect, therefore, the present invention provides a process for preparing a compound of general formula (I) or a pharmaceutically acceptable salt thereof, which comprises:
a) reacting a compound of general formula (II) 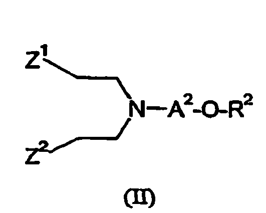 in which each of Z¹ and Z² independently represents a leaving atom or group, or a corresponding compound in which one or more substituents on R² are protected, with a compound of general formula (III)

   R¹-O-A¹-NH₂ (III)
b) reacting a compound of general formula (IV) 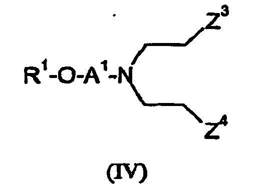 in which each of Z³ and Z⁴ independently represents a leaving atom or group, with a compound of general formula (V)

   H₂N-A²-O-R² (V)

   or a corresponding compound in which one or more substituents on R² are protected;
c) reacting a compound of general formula (VI) 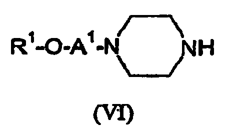 with a compound of general formula (VII)

   Z-A²-O-R² (VII)

   in which Z² represents a leaving atom or group;
d) reacting a compound of formula (VIII) 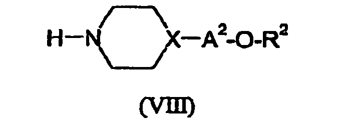 with a compound of general formula (IX)

   R¹-O-A¹-Z⁶ (IX)

   in which Z⁶ represents a leaving atom or group; or
e) for a compound of formula (I) in which A¹ represents (CH₂)ₘ and A² represents (CH₂)ₙ, reducing a corresponding compound of formula (I) in which A¹ represents (CH₂)ₘ₋₁C(=O) and/or A² represents C(=O)(CH₂)ₙ₋₁;
followed by removing any protecting group and, optionally, forming a pharmaceutically acceptable salt.

Referring to process step a), the leaving atoms or groups represented by Z¹ and Z² may be, for example, hydrocarbylsulfonyloxy groups, such as methanesulfonyloxy or p-toluenesulfonyloxy, or halogen atoms, such as chlorine atoms.

The reaction is conveniently performed at a temperature in the range of from 0 to 100 °C, such as from 50 to 90 °C. Convenient solvents include organic solvents, for example amides such as dimethylformamide. The reaction is conveniently performed in the presence of a base, for example an alkali metal carbonate such as potassium carbonate. The reaction may be performed in the presence of a catalyst, such as sodium iodide.

Compounds of general formula (III) can be prepared from the corresponding compounds of general formula (X) 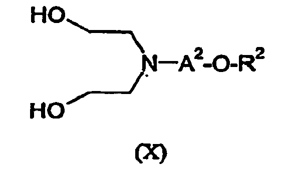 for example by reaction with thionyl chloride to afford a compound of formula (III) in which Z¹ and Z² represent chlorine atoms.

Compounds of general formula (X) can be prepared by reacting a compound of general formula (XI) 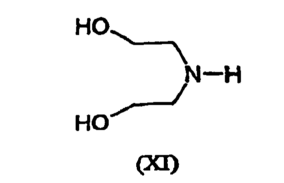 with a compound of general formula (VII), analogous to process c) described herein.

Process step b) may be performed by a method analogous to that described herein for process step a). The requisite starting materials may be obtained by reacting a compound of formula (XI) with a compound of formula (IX), then, for example, reacting the resultant diol with thionyl chloride to afford a compound of formula (IV).

Referring to process c), depending upon whether or not A² contains a carbonyl group, the leaving atom or group represented by Z⁵ may be, for example, a hydroxyl group, an acyloxy group or a halogen atom, such as a bromine atom. The reaction is conveniently performed at a temperature in the range of from 0 to 100 °C. Convenient solvents include organic solvents, for example amides such as dimethylformamide. When the compound of formula (VII) is an alkyl halide, the reaction is conveniently performed in the presence of a base, for example an alkali metal carbonate such as caesium carbonate. When Z⁵ represents a hydroxyl group, the reaction is conveniently conducted under amide-bond coupling conditions, for example in the presence of 1-hydroxybenzotriazole and dicyclohexylcarbodiimide.

Compounds of formula (VI) may be prepared by deprotecting a compound of formula (XII) 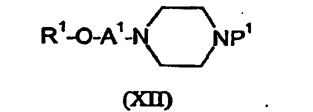 in which P¹ represents an amino protecting group, such as t-butoxycarbonyl. For example, a t-butoxycarbonyl group may be removed using trifluoroacetic acid. Alternatively, when the compound of formula (I) is symmetrical, it may be prepared *in situ* by reacting piperazine with two equivalents of a compound of formula (VII) and allowing the reaction to go to completion.

Process d) may be performed by the same method as process c).

Referring to process step e), the reducing agent can conveniently be a borane (BH₃), a borohydride reducing agent, such as sodium borohydride, or an alkali metal aluminium hydride, such as lithium aluminium hydride. The reduction is conveniently performed in the presence of a solvent such as an ether, for example tetrahydrofuran. The temperature at which the reduction is carried out is conveniently in the range of from -25 to 100 °C, such as from -10 to 40 °C.

A pharmaceutically acceptable salt may be formed by a conventional method, such as by reacting a compound of formula (I) with a pharmaceutically acceptable acid, such as hydrochloric acid.

Certain of the intermediates may be noveL The invention also provides all the novel intermediates disclosed herein.

The compounds of the invention may be administered by any convenient route, e.g. into the gastrointestinal tract (e.g. rectally or orally), the nose, lungs, musculature or vasculature or transdermally. The compounds may be administered in any convenient administrative form, e.g. tablets, powders, capsules, solutions, dispersions, suspensions, syrups, sprays, suppositories, gels, emulsions, patches etc. Such compositions may contain components conventional in pharmaceutical preparations, e.g. diluents, carriers, pH modifiers, sweeteners, bulking agents, and further active agents. If parenteral administration is desired, the compositions will be sterile and in a solution or suspension form suitable for injection or infusion. Such compositions form a further aspect of the invention.

According to another aspect, the present invention provides a pharmaceutical composition, which comprises a compound of formula (I) or a pharmaceutically acceptable salt thereof, as defined hereinabove, together with a pharmaceutically acceptable diluent or carrier.

According to yet another aspect, the present invention provides a compound of formula (I), or a pharmaceutically acceptable salt thereof, as defined hereinabove, for use in therapy.

According to another aspect, the present invention provides the use of a compound of formula (I) 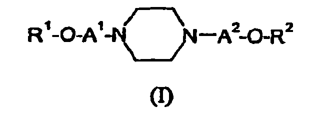 in which:-
R¹ represents a phenyl group that is substituted by one or two fluorine atoms;
A¹ represents (CH₂)ₘ or (CH₂)ₘ₋₁C(=O);
m is 2, 3, 4 or 5;
A² represents (CH₂)ₙ or C(=O)(CH₂)ₙ₋₁;
n is 2, 3, 4 or 5; and
R² represents a phenyl group that is substituted by one, two or three substituents selected independently from (1-2C)alkylenedioxy, a hydroxyl group, a cyano group, a (3-6C)cycloalkyl group, a halo(1-4C) alkyl group, a (1-4C)alkoxy group, a (1-4C)alkoxy(1-4C)alkoxy group, a hydroxy(1-4C)alkoxy group, a (1-4C)alkylsulfanyl group, a (1-4C)alkylsulfonyl group, a halo(1-4C)alkoxy group and a halo(1-4C)alkylsulfonyl group;
or a pharmaceutically acceptable salt thereof, in the manufacture of a medicament for the treatment of a neurological disorder or a psychiatric disorder that has been linked to sigma receptors.

According to yet another aspect, the present invention provides a a compound of general formula (I) 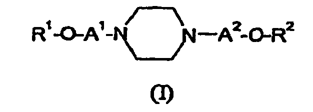 in which:-
R¹ represents a phenyl group that is substituted by one or two fluorine atoms;
A¹ represents (CH₂)ₘ or (CH₂)ₘ₋₁C(=O);
m is 2, 3,4 or 5;
A² represents (CH₂)ₙ or C(=O)(CH₂)ₙ₋₁;
n is 2, 3, 4 or 5; and
R² represents a phenyl group that is substituted by one, two or three substituents selected independently from (1-2C)alkylenedioxy, a hydroxyl group, a cyano group, a (3-6C)cycloalkyl group, a halo(1-4C) alkyl group, a (1-4C)alkoxy group, a (1-4C)alkoxy(1-4C)alkoxy group, a hydroxy(1-4C)alkoxy group, a (1-4C)alkylsulfanyl group, a (1-4C)alkylsulfonyl group, a halo(1-4C)alkoxy group and a halo(1-4C)alkylsulfonyl group;
or a pharmaceutically acceptable salt thereof, for treating a neurological disorder or a psychiatric disorder that has been linked to sigma receptors, or a patient.

The subject may be a human or a non-human animal, such as a non-human mammal, for example a cat, dog, horse, cow or sheep. Preferably the subject is a human.

The disorder responsive to a sigma receptor modulator may be, for example, a disorder of the central nervous system, such as a neurological disorder or a psychiatric disorder that has been linked to sigma receptors. Examples of neurological disorders include cerebral deficits subsequent to cardiac bypass surgery and grafting, cerebral ischemia (e.g. associated with stroke or cardiac arrest); spinal cord trauma; head trauma; multiple sclerosis, Alzheimer's Disease; Huntington's Chorea; amyotrophic lateral sclerosis; AIDS-induced dementia; muscular spasms; convulsions; drug tolerance, withdrawal, and cessation (i.e. opiates, benzodiazepines, nicotine, cocaine, or ethanol); ocular damage and retinopathy; cognitive disorders; idiopathic and drug-induced Parkinson's Disease; pain; and movement disorders such as tardive dyskinesia. Examples of psychiatric disorders that are treated with a compound of formula I include schizophrenia, anxiety and related disorders (e.g. panic attack and stress-related disorders), depression, bipolar disorders, psychosis, and obsessive compulsive disorders.

The compounds according to the invention are of particular interest for use as neuroprotective agents and in the treatment of patients to facilitate neuronal regeneration and functional recovery after onset of a neurodegenerative disease, in particular ischemic stroke, traumatic brain injury, spinal chord injury and multiple sclerosis.

The dosage of the compounds of formula (I) will depend upon the nature and severity of the condition being treated, the administration route and the size and species of the subject. In general, quantities in the range of from 0.01 to 100 mg/kg bodyweight will be administered.

As used herein, the term "treatment" includes prophylactic use. The term "effective amount" refers to the amount of the compound of formula (I) that is effective to reduce or inhibit the development of the symptoms of the disorder being treated.

The compound according to the invention may be administered alone or in combination with another therapeutic agent having a different mode of action.

The ability of a compound to bind to a sigma receptor may be demonstrated by one or more of the following tests.

Sigma-1 (σ1) and sigma-2 (σ2) receptor binding assays are carried out in membranes from HEK-293 (Human Embryonic Kidney) cells.

### Membrane preparation:

Confluent HEK-293 cells are harvested in PBS/5 mM EDTA. They are centrifuged at 2000 rpm for 5 min and then washed two times in PBS. Cells are homogenized in 20 mM Tris-HCL (pH = 7.5) containing 5 mM EDTA, 0.5 mM PMSF and 0.5 µg/mL leupeptin using a Dounce homogenizer and sonicated for 5 minutes.

Nuclear debris and intact cells are removed by centrifugation at 3000 rpm for 10 minutes at 4 °C. The supernatant is centrifuged at 12000 rpm for 30 minutes and the resulting pellet is resuspended in 25 mM Tris-HCL (pH = 7.5), 25 mM Mg₂Cl, 10% sucrose containing 0.5 mM PMSF, 2 mM AEBSF, 1 mM EDTA, 130 µM bestatin, 14 µM E-64, 1 µM leupeptin and 0.3 mM aprotinin.

Proteins are determined using the Bio Rad Protein Assay Dye Reagent and the membranes are aliquoted and frozen at -80°C.

### σ1 receptor binding assay

The binding assays are performed in 96-well plates.

σ1 receptors are labeled using the σ1 selective probe (+) - [³H] Pentazocine (Bowen WD et al, Mol Neuropharmacol 3, 117-126, 1993).

Total binding is determined by incubating 50 µg of HEK-293 cell membranes with 10 nM (+)- [³H]-pentazocine (Perkin-Elmer, 35 Ci/mmol) and assay buffer (50 mM Tris-HCl, pH = 8.3) in a total volume of 200 µl. Non specific binding is determined in the presence of 10 µM unlabeled pentazocine. For competition experiments, 50 µl of displacing compound is added at 8 different concentrations. Incubations are carried out for 120 min at 37°C. Assays are terminated by dilution with ice-cold 10 mM Tris-HCl, pH = 8.3 and vacuum filtration through glass fibers using a Skatron cell harvester from Molecular Devices. The filters are washed three times and the membrane-bound radioactivity is determined in a Microbeta scintillation counter.

Filters are soaked in 0.5 % polyethyleneimine for 1 hour before use.

Specific binding is determined by subtraction of non specific binding from total binding. IC₅₀ values (concentration of competing ligand required for 50% inhibition of [³H]-pentazocine binding) are analyzed by non-linear regression fit using the GraphPad Prism software.

### σ2 receptor binding assay

The binding assays are performed in 96-well plates

σ2 receptors are labeled using [³H] DTG (Di-o-tolylguanidine), under conditions in which σ1 receptors are masked with the σ1 selective compound pentazocine (Hellewell SB et al, Eur. J. Pharmacol, 268, 9-18, 1994).

Total binding is determined by incubating 50 µg of HEK-293 cell membranes with 10 nM [³H]-DTG (Perkin-Elmer, 58 Ci/mmol) in the presence of 10 µM pentazocine and assay buffer (50 mM Tris-HCl, pH = 8.3) in a total volume of 200 µl. Non specific binding is determined in the presence of 10 µM unlabeled DTG. For competition experiments, 50 µl of displacing compound is added at 8 different concentrations. Incubations are carried out for 120 min at 37°C. Assays are terminated by dilution with ice-cold 10 mM Tris-HCl, pH = 8.3 and vacuum filtration through glass fibers using a Skatron cell harvester from Molecular Devices. The filters are washed three times and the membrane-bound radioactivity is determined in a Microbeta scintillation counter.

Filters are soaked in 0.5 % polyethyleneimine for 1 hour before use.

Specific binding is determined by subtraction of non specific binding from total binding. IC₅₀ values (concentration of competing ligand required for 50% inhibition of [³H]-DTG binding) are analyzed by non-linear regression fit using the GraphPad Prism software

The known compound 1,4-bis-[2-(4-fluorophenoxy)ethyl]piperazine and the compound of Reference Example 1 have both been found to have an IC₅₀ of less than 700 nM in the σ1 receptor binding assay.

The following examples illustrate the invention.

### Reference Example 1

### 1,4-Bis-[2-(2-fluorophenoxy)ethyl]piperazine dihydrochloride

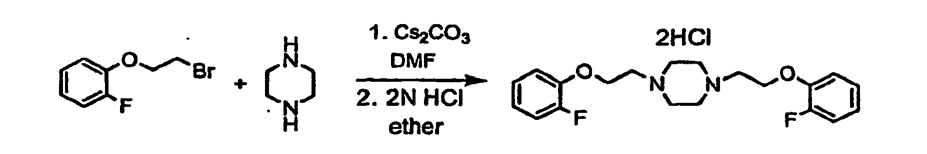

To piperazine (0.29 g, 3 mmol) in DMF (4 ml) was added Cs₂CO₃ (4.4 g, 13.5 mmol) and 1-(2-bromo-ethoxy)-2-fluoro-benzene (1.5 g, 6.8 mmol). The mixture was stirred at room temperature for a day, quenched with water, and extracted with dichloromethane. The organic layer was concentrated to give a white solid (0.88 g, 81%). Part of this solid (0.2 g) was suspended in ether and 2N HCl in ether (1 ml). The white solid was filtered to give the title compound (0.23 g, 96%).
¹H-NMR (400MHz, CD₃OD-d₄): δ 3.70-3.76 (m, 12H), 4.49 (t, 4H), 7.03 (m, 2H), 7.18 (m, 6H).

The following compounds were prepared using the same method as descried in Reference Example 1.

### Reference Examp!e 2

### 1,4-Bis-[3-(4-fluorophenoxy)propyl]piperazine

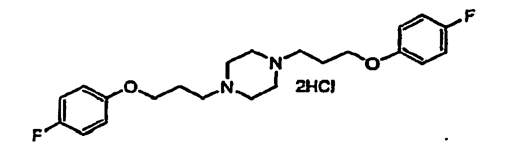

¹H-NMR (400MHz, CD₃OD-d₄): δ 2.29 (m, 4H), 3.5-4.0 (m, 12H), 4.11 (t, 4H), 6.95 (m, 4H), 7.02 (m, 4H).

### Reference Example 3

### 1,4-Bis-[3-(2-fluorophenoxy)propyl]piperazine dihydrochloride

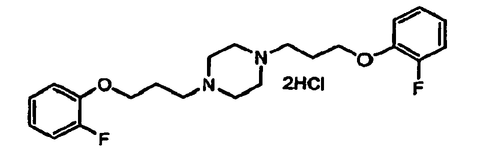

¹H-NMR (400MHz, CD₃OD-d₄): δ 2.36 (m, 4H), 3.5-4.0 (m, 12H), 4.23 (t, 4H), 6.96 (m 2H), 7.12 (m, 6H).

### Reference Example 4

### 1-[2-(4-Fluorophenoxy)ethyl]-4-[3-[4-floorophenoxy)propyl])piperazine dihydrochloride

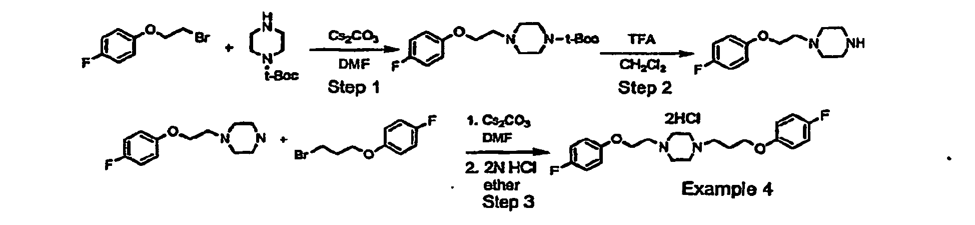

### Step 1: 4-[2-(4-Fluorophenoxy)ethyl]pipemine-1-carboxylic acid tert-butyl ester

To a solution of piperazine-1-carboxylic acid *tert*-butyl ester (1.86 g, 10 mmol) and 1-(2-bromoethoxy)-4-fluorobenzene (2.19 g, 10 mmol) in DMF (5 ml) was added Cs₂CO₃ (7.5 g, 20 mmol). The mixture was stirred at room temperature for a day, then quenched with water, and extracted with ethyl acetate. The organic layer was washed with water and brine, dried over anhydrous sodium sulphate, and concentrated to yield the title compound quantitatively as an oil (3.25 g) which was used without further purification.

¹H-NMR (400MHz, DMSO-d₆): δ 1.39 (s, 9H), 2.42 (t, 4H), 2.69 (t, 2H), 3.31 (t, 4H), 4.04 (t, 2H), 6.95 (m, 2H), 7.10 (t, 2H).

### Step 2: 1-[2-(2-Fluorophenoxy)ethyl)ethyl]piperazine

To the solution of 4-[2-(4-fluorophenoxy)ethyl]piperazine-1-carboxylic acid *tert*-butyl ester (3.2 g, 10 mmol) in dichloromethane (80 ml) was added with trifluoroacetic acid (15 ml). The reaction mixture was stirred at room temperature for 2 h and concentrated. The residue was treated with a saturated solution of NaHCO₃ extracted with dichloromethane. The organic layer was collected and concentrated to give the title compound quantitatively (5.0 g) as a white solid.

¹H-NMR (400MHz, DMSO-d₆): δ 2.35 (m, 10H), 4.13 (t, 2H), 7.01 (t, 2H), 7.16 (t, 2H), 9.20 (b, 1H).

### Step 3: 1-[2-(4-Fluorophenoxy)ethyl]-4-[3-(4-fluorophenoxy)propyl]piperazine dihydrochloride

To 1-[2-(4-fluorophenoxy)ethyl]piperazine (0.148 mg, 0.5 mmol) in DMF (3 ml) was added Cs₂CO₃ (0.8 g, 2.5 mmol) and 1-(3-bromopropoxy)-4-fluorobenzene (139 mg, 0.6 mmol). The mixture was stirred at room temperature for a day, quenched with water and extracted with dichloromethane. The organic layer was concentrated and the residue was suspended in ether and 2N HCl in ether (2 ml). The white solid was filtered out to give the title compound (171 mg, 76%).

¹H-NMR (400MHz, CD₃OD-d₄): 5 2.36 (m, 2H), 3.55 (t, 2H), 3.5-4.0 (b, 10H), 4.22 (t, 2H), 4.42 (t, 2H), 7.05 (m, 8H).

### Reference Example 5 2-(4-Fluorephenoxy)-1-{4-[2-(4-fluorophenoxy)acetyl]piperazin-1-yl}-ethanone

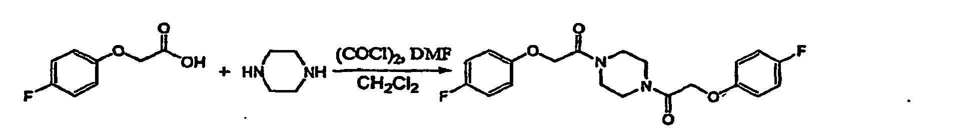

To the solution of (4-fluorophenoxy)acetic acid (8.5 g, 0.05 mol) in anhydrous dichloromethane (120 ml) was added oxalyl chloride (8.9 ml, 0.1mol) and DMF (0.02 ml). The reaction mixture was stirred at room temperature for 1 h and concentrated. The residue was dissolved in anhydrous dichloromethane (60 ml). To this solution was added piperazine (1.9 g, 22 mmol) and triethylamine (11 ml, 0.08 mol). The reaction mixture was stirred overnight. The precipitate was filtered out, washed with methanol, and dried to afford the title compound quantitatively (9.16 g) as a white solid.

¹H-NMR 400MHz, (DMSO-d₆): δ 5.48 (d, 8H), 4.85 (s, 4H), 6.95 (m, 4H), 7.11(m, 4H)

The following compound and their pharmaceutically acceptable salts can be prepared using the same method as descried in Reference Example 1.
4-(2-{4-[2-(4-Fluorophenoxy)ethyl]-piperazin-1-yl}ethoxy)benzonitrile
4-(2-{4-[2-(2-Fluorophenoxy)ethyl]piperazin-1-yl}ethoxy)benzonitrile
4-(2-{4-[2-(2,4-Difluorophenoxy)ethyl]piperazin-1-yl}ethoxy)benzonitrile
3-(2-{4-[2-(4-Fluorophenoxy)ethyl]piperazin-1-yl}ethoxy)benzonitrile
3-(2-{4-[2-(2-Fluorophenoxy)ethyl]piperazin-1-yl}ethoxy)benzonitrile
3-(2-{4-[2-(2,4-Difluorophenoxy)ethyl]piperazin-1-yl}ethoxy)benzonitrile
1-[2-(4-Fluorophenoxy)ethyl]-4-[2-(3-trifluoromethylphenoxy)ethyl]piperazine
1-[2-(2-Fluorophenoxy)ethyl]-4-[2-(3-trifluoromethylphenoxy)ethyl]piperazine
1-[2-(2,4-Difluorophenoxy)ethyl]-4-[2-(3-trifluoromethylphenoxy)ethyl]ppiperizine
1-[2-(4-Fluorophenoxy)ethyl]-4-[2-(4-trifluoromethylphenoxy)ethyl]piperazine
1-[2-(2-Fluorophenoxy)ethyl]-4-[2-(4-fluoromethylphenoxy)ethyl]piperazine
1[(2-(2,4-Difluorophenoxy)ethyl]-4-[2-(4-trifluoromethylphenoxy)ethyl]piperazine
1-[2-(4-Fluoropheaoxy)ethyl]-4-[2-(4-methanesulfonylphenoxy)ethyl]piperazine
1-[2-(2-Fluorophenoxy)ethyl]-4-[2-(4-methanesulfonylphenoxy)ethyl]piperazine
1-[2-(2,4-Difluorophenoxy)ethyl]-4-[2-(4-methanesulfonylphenoxy)ethyl]piperazine
1-[2-(4-Fluorophenoxy)ethyl]-4-[2-(3-methanesulfonylphenoxy)ethyl]piperazine
1-[2-(2-Fluorophenoxy)ethyl]-4-[2-(3-methanesulfonylphenoxy)ethyl]piperazine
1-[2-(2,4-Difluorophenoxy)ethyl]-4-[2-(3-methanesulfonylphenoxy)ethyl]piperazine
1-[2-(4-Fluolophenoxy)ethyl]-4-[2-(4-trifluoromethanesulfonylphenoxy)ethyl]piperazine
1-[2-(2-Fluorophenoxy)ethyl]-4-[2-(4-trifluoromethanesulfonylphenoxy)ethyl]piperazine
1-[2-(2,4-Difluorophenoxy)ethyl]-4-[2-(4-trifluoromethanesulfonylphenoxy)ethyl]piperazine
1-[2-(4-Fluorophenoxy)ethyl]4-[2-(3-trifluoromethanesulfonylphenoxy)ethyl]piperazine
1-[2-(2-Fluorophenoxy)ethyl]-4-[2-(3-trifluommethanesulfonylphenoxy)ethyl]piperazine
1-[2-(2,4-Difluorophenoxy)ethyl]-4-[2-(3-trifluoromethanesulfonylphenoxy)ethyl]piperazine
4-(3-{4-[2-(4-Fluorophenoxy)ethyl]piperazin-1-yl}propoxy)benzonitrile
4-(3-{4-[2-(2-Fluorophenoxy)ethyl]piperazin-l-yl}propoxy)benzonibile
4-(3-{4-[2-(2,4-Difluophenoxy)ethyl]piperazin-1-yl}propoxy)benzonitrile
3-(3-{4-[2-(4-Fluorophenoxy)ethyl]piperazin-1-yl}propoxy)benzonitrile
3-(3-{4-[2-(2-Fluorophenoxy)ethyl]piperazin-1-yl}propoxy)benzonitrile
3-(3-{4-[2-(2,4-Difluorophenoxy)ethyl]piperazin-1-yl}propoxy)benzonitrile
1-[2-(4-Fluorophenoxy)ethyl]-4-[3-(4-trifluoromethylphenoxy)propyl]piperazine
1-[2-(2-Fluorophenoxy)ethyl]-4-[3-(4-trifluoromethylphenoxy)propyl]piperazine
1-[2-(2,4-Difluorophenoxy)ethyl]-4-[3-(4-trifluoromethylphenoxy)propyl]piperazine
1-[2-(4-Fluorophenoxy)ethyl]-4-[3-(3-trifluoromethylphenoxy)propyl]piperazine
1-[2-(2-Fluorophenoxy)ethyl]-4-[3-(3-trifluoromethylphenoxy]propyl]piperazine
1-[2-(2,4-Difluorophenoxy)-ethyl]-4-[3-(3-trifluoromethylphenoxy)propyl]piperazine
1-[2-(4-Fluorophenoxy)ethyl]-4-[3-(4-methanesulfonylphenoxy)propyl]piperazine
1-[2-(2-Fluorophenoxy)ethyl]-4-[3-(4-methanesulfonylphenoxy)propyl]piperazine
1-[2-(2,4-Difluorophenoxy)ethyl]-4-[3-(4-methanesulfonylphenoxy)propyl]piperazine
1-[2-(4-Fluorophenoxy)ethyl]-4-[3-(3-methanesulfonylphenoxy)propyl]piperazine
1-[2-(2-Fluorophenoxy)ethyl]-4-[3-(3-methanesulfonylphenoxy)propyl]piperazine
1-[2-(2,4-Difluorophenoxy)ethyl]-4-[3-(3-methanesulfonylphenoxy)propyl]piperazine
1-[2-(4-Fluorophenoxy)ethyl]-4-[3-(4-trifluoromethanesulfonylphenoxy)propyl]piperazine
1-[2-(2-Fluorophenoxy)ethyl]-4-[3-(4-trifluoromethanesulfonylphenoxy]propyl]piperazine
1-[2-(2,4-Difluorophenoxy)ethyl]4-[3-(4-trifluoromethanesulfonylphenoxy)propyl]-piperazine
1-[2-(4-Fluorophenoxy)ethyl]-4-[3-(3-trifluoromethanesulfonylphenoxy)propyl]piperazine
1-[2-(2-Fluorophenoxy)ethyl]-4-[3-(3-trifluoromethanesulfonylphenoxy)propyl]piperazine; and
1-[2-(2,4-Difluorophenoxy)ethyl]-4-[3-(3-trifluoromethanesulfonylphenoxy)propyl]-piperazine.

## Claims

1. A compound of general formula (I) 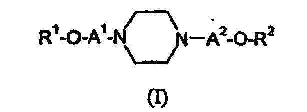 in which:-
R¹ represents a phenyl group that is substituted by one or two fluorine atoms;
A¹ represents (CH₂)ₘ or (CH₂)ₘ₋₁C(=O);
m is 2,3,4 or 5;
A² represents (CH₂)ₙ or C(-O)(CH₂)ₙ₋₁;
n is 2, 3, 4 or 5; and
R² represents a phenyl group that is substituted by one, two or three substituents selected independently from (1-2C)alkylenedioxy, a hydroxyl group, a cyano group, a (3-6C)cycloalkyl group, a halo(1-4C) alkyl group, a (1-4C)alkoxy group, a (1-4C)alkoxy(1-4C)alkoxy group, a hydroxy(1-4C)alkoxy group, a (1-4C)alkylsulfanyl group, a (1-4C)alkylsulfonyl group, a halo(1-4C)alkoxy group and a halo(1-4C)alkylsulfonyl group;
or a pharmaceutically acceptable salt thereof.

2. A compound as claimed in Claim 1, in which R¹ represents a 2-fluorophenyl, 3-fluorophenyl, 4-fluorophenyl or 2,4-difluorophenyl group.

3. A compound as claimed in Claim 1 or Claim 2, in which m is 2 or 3.

4. A compound as claimed in Claim 3, in which m is 2.

5. A compound as claimed in any one of Claims 1 to 4, in which A' represents (CH₂)ₘ.

6. A compound as claimed in any one of Claims 1 to 5, in which n is 2 or 3.

7. A compound as claimed in any one of Claims 1 to 6, in which A² represents (CH₂)ₙ.

8. A compound as claimed in any one of Claims 1 to 7, in which R² represents a phenyl group that is substituted by one or two substituents selected independently from a cyano group, a fluoro(1-4C) alkyl group, a (1-4C)alkylsulfonyl group, a fluoro(1-4C)alkoxy group and a fluoro(1-4C)alkylsulfonyl group.

9. A compound as claimed in Claim 8, in which R² represents 3-cyanophenyl, 4-cyanophenyl, 3-trifluoromethylphenyl, 4- trifluoromethylphenyl, 3-methylsulfonylphenyl, 4-methylsulfonylphenyl, 3-trifluoromethylsulfonylphenyl or 4-trifluoromethylsulfonylphenyl.

10. A process for preparing a compound as claimed in any one of Claims 1 to 9, which comprises
a) reacting a compound of general formula (II) 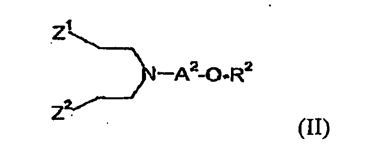 in which each of Z¹ and Z² independently represents a leaving atom or group, or a corresponding compound in which one or more substituents on R² are protected, with a compound of general formula (III)
R¹-O-A¹-NH₂ (III)
b) reacting a compound of general formula (IV) 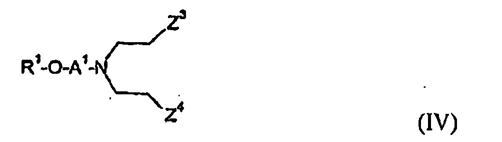 in which each of Z³ and Z⁴ independently represents a leaving atom or group, with a compound of general formula (V)
H₂N-A²-O-R² (V)
or a corresponding compound in which one or more substituents on R² are protected;
c) reacting a compound of general formula (VI) 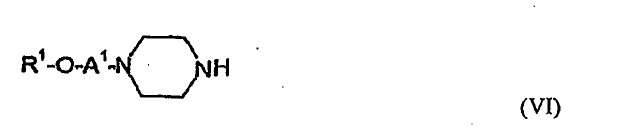 with a compound of general formula (VII)
Z⁵-A²-O-R² (VII)
in which Z⁵ represents a leaving atom or group;
d) reacting a compound of formula (VIII) 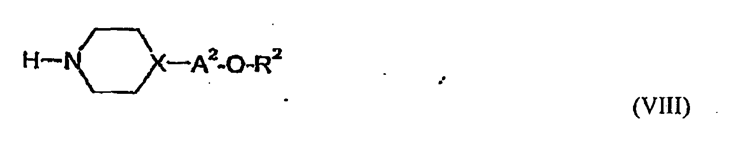 with a compound of general formula (IX)
R¹=O-A¹-Z⁶ (IX)
in which Z⁶ represents a leaving atom or group; or
e) for a compound of formula (I) in which A¹ represents (CH₂)ₘ and A² represents (CH²)ₙ, reducing a corresponding compound of formula (I) in which A¹ represents (CH₂)ₘ₋₁C(=O) and/or A² represents C(=O)(CH₂)ₙ₋₁;
followed by removing any protecting group and, optionally, forming a pharmaceutically acceptable salt.

11. A pharmaceutical composition, which comprises a compound as claimed in any one of claims 1 to 9, and a pharmaceutically acceptable diluent or carrier.

12. A compound of general formula (I) 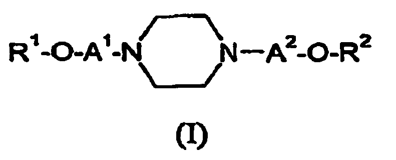 in which:-
R¹ represents a phenyl group that is substituted by one or two fluorine atoms;
A¹ represents (CH₂)ₘ or (CH₂)ₘ₋₁C(=O);
m is 2, 3, 4 or 5;
A² represents (CH₂)ₙ or C(=O)(CH₂)ₙ₋₁;
n is 2, 3,4 or 5; and
R² represents a phenyl group that is substituted by one, two or three substituents selected independently from (1-2C)alkylenedioxy, a hydroxyl group, a cyano group, a (3-6C)cycloalkyl group, a halo(1-4C) alkyl group, a (1-4C)alkoxy group, a (1-4C)alkoxy(1-4C)alkoxy group, a hydroxy(1-4C)alkoxy group, a (1-4C)alkylsulfanyl group, a (1-4C)alkylsulfonyl group, a halo(1-4C)alkoxy group and a halo(1-4C)alkylsulfonyl group;
or a pharmaceutically acceptable salt thereof,
for use in a method of treating a neurological disorder or a psychiatric disorder that has been linked to sigma receptors in a patient.

13. A compound of claim 12, wherein the neurological disorder is selected from cerebral deficits subsequent to cardiac bypass surgery and grafting, cerebral ischemia; spinal cord trauma; head trauma; multiple sclerosis, Alzheimer's Disease; Huntington's Chorea; amyotrophic lateral sclerosis; AIDS-induced dementia; muscular spasms; convulsions; drug tolerance, withdrawal, and cessation; ocular damage and retinopathy; cognitive disorders; idiopathic and drug-induced Parkinson's Disease; pain; and movement disorders such as tardive dyskinesia, and the psychiatric disorder is selected from schizophrenia, anxiety and related disorders, depression, bipolar disorders, psychosis, and obsessive compulsive disorders.

14. A compound as claimed in any one of Claims 1 to 9, for use in therapy.

15. Use of a compound of general formula (I) 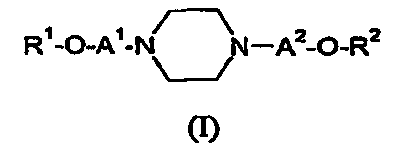 in which:-
R¹ represents a phenyl group that is substituted by one or two fluorine atoms;
A¹ represents (CH₂)ₘ or (CH₂)ₘ₋₁C(=O);
m is 2, 3, 4 or 5;
A² represents (CH₂)ₙ or C(=O)(CH₂)ₙ₋₁ ;
nis2,3,4or5;and
R² represents a phenyl group that is substituted by one, two or three substituents selected independently from (1-2C)alkylenedioxy, a hydroxyl group, a cyano group, a (3-6C)cycloalkyl group, a halo(1-4C) alkyl group, a (1-4C)alkoxy group, a (1-4C)alkoxy(1-4C)alkoxy group, a hydroxy(1-4C)alkoxy group, a (1-4C)alkylsulfanyl group, a (1-4C)alkylsulfonyl group, a halo(1-4C)alkoxy group and a halo(1-4C)alkylsulfonyl group;
or a pharmaceutically acceptable salt thereof, in the manufacture of a medicament for the treatment of a neurological disorder or a psychiatric disorder that has been linked to sigma receptors.

16. The use of claim 15 wherein the neurological disorder is selected from cerebral deficits subsequent to cardiac bypass surgery and grafting, cerebral ischemia; spinal cord trauma; head trauma; multiple sclerosis, Alzheimer's Disease; Huntington's Chorea; amyotrophic lateral sclerosis; AIDS-induced dementia; muscular spasms; convulsions; drug tolerance, withdrawal, and cessation; ocular damage and retinopathy; cognitive disorders; idiopathic and drug-induced Parkinson's Disease; pain; and movement disorders such as tardive dyskinesia, and the psychiatric disorder is selected from schizophrenia, anxiety and related disorders, depression, bipolar disorders, psychosis, and obsessive compulsive disorders.

## Patentansprüche

1. Verbindung der allgemeinen Formel (I) 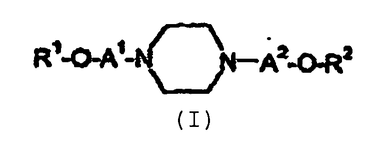 worin:
R¹ für eine Phenylgruppe steht, die durch ein oder zwei Fluoratome substituiert ist;
A¹ für (CH₂)ₘ oder (CH₂)ₘ₋₁C(=O) steht;
m für 2, 3, 4 oder 5 steht;
A² für (CH₂)ₙ oder C (=O) (CH₂) ₙ₋₁ steht;
n für 2, 3, 4 oder 5 steht und
R² für eine Phenylgruppe steht, die durch einen, zwei oder drei unabhängig voneinander unter C₁₋₂-Alkylendioxy, einer Hydroxylgruppe, einer Cyanogruppe, einer C₃₋₆-Cycloalkylgruppe, einer Halogen-C₁₋₄-alkylgruppe, einer C₁₋₄-Alkoxygruppe, einer C₁₋₉-Alkoxy-C₁₋₉-alkoxygruppe, einer Hydroxy-C₁₋₄-alkoxygruppe, einer C₁₋₄-Alkylsulfanylgruppe, einer C₁₋₉-Alkylsulfonylgruppe, einer Halogen-C₁₋₄-alkoxygruppe und einer Halogen-C₁₋₄-alkylsulfonylgruppe ausgewählte Substituenten substituiert ist;
oder ein pharmazeutisch unbedenkliches Salz davon.

2. Verbindung nach Anspruch 1, worin R¹ für eine 2-Fluorphenyl-, 3-Fluorphenyl-, 4-Fluorphenyl- oder 2,4-Difluorphenylgruppe steht.

3. Verbindung nach Anspruch 1 oder 2, worin m für 2 oder 3 steht.

4. Verbindung nach Anspruch 3, worin m für 2 steht.

5. Verbindung nach einem der Ansprüche 1 bis 4, worin A¹ für (CH₂)ₘ steht.

6. Verbindung nach einem der Ansprüche 1 bis 5, worin n für 2 oder 3 steht.

7. Verbindung nach einem der Ansprüche 1 bis 6, worin A² für (CH₂)ₘ steht .

8. Verbindung nach einem der Ansprüche 1 bis 7, worin R² für eine Phenylgruppe steht, die durch einen oder zwei unabhängig voneinander unter einer Cyanogruppe, einer Fluor-C₁₋₄-alkylgruppe einer C₁₋₄-Alkylsulfonylgruppe, einer Fluor-C₁₋₄-alkoxygruppe und einer Fluor-C₁₋₄-alkylsulfonylgruppe ausgewählte Substituenten substituiert ist.

9. Verbindung nach Anspruch 8, worin R² für 3-Cyanophenyl, 4-Cyanophenyl, 3-Trifluormethylphenyl, 4-Trifluormethylphenyl, 3-Methylsulfonylphenyl, 4-Methylsulfonylphenyl, 3-Trifluormethylsulfonylphenyl oder 4-Trifluormethylsulfonylphenyl steht.

10. Verfahren zur Herstellung einer Verbindung nach einem der Ansprüche 1 bis 9, bei dem man
a) eine Verbindung der allgemeinen Formel (II) 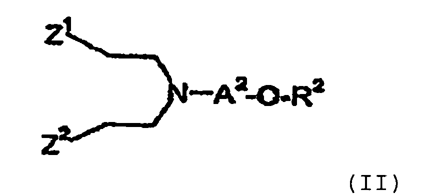 worin Z¹ und Z² jeweils unabhängig voneinander für ein Abgangsatom oder eine Abgangsgruppe stehen, oder eine entsprechende Verbindung, in der ein oder mehrere Substituenten an R² geschützt sind, mit einer Verbindung der allgemeinen Formel (III)
R¹-O-A¹-NH₂ (III)
umsetzt;
b) eine Verbindung der allgemeinen Formel (IV) 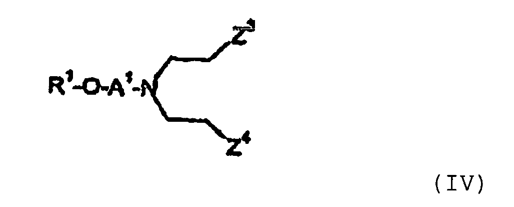 worin Z³ und Z⁴ jeweils unabhängig voneinander für ein Abgangsatom oder eine Abgangsgruppe stehen, mit einer Verbindung der allgemeinen Formel (V)
H₂N-A²-O-R² (V)
oder einer entsprechenden Verbindung, in der ein oder mehrere Substituenten an R² geschützt sind, umsetzt;
c) eine Verbindung der allgemeinen Formel (VI) 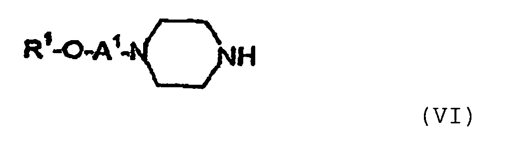 mit einer Verbindung der allgemeinen Formel (VII)
Z⁵-A²-O-R² (VII)
worin Z⁵ für ein Abgangsatom oder eine Abgangsgruppe steht, umsetzt;
d) eine Verbindung der allgemeinen Formel (VIII) 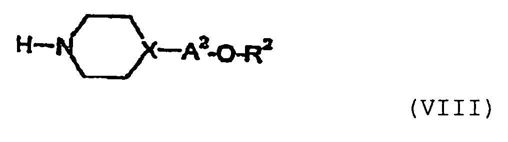 mit einer Verbindung der allgemeinen Formel (IX)
R¹-O-A¹-Z⁶ (IX)
worin Z⁶ für ein Abgangsatom oder eine Abgangsgruppe steht, umsetzt; oder
e) für eine Verbindung der Formel (I), in der A¹ für (CH₂)ₘ steht und A² für (CH₂)ₙ steht, eine entsprechende Verbindung der Formel (I), in der A¹ für (CH₂) ₘ₋₁C (=O) steht und/oder A² für (C=O) (CH₂) ₙ₋₁ steht, reduziert;
und danach jegliche Schutzgruppen abspaltet und gegebenenfalls ein pharmazeutisch unbedenkliches Salz bildet.

11. Pharmazeutische Zusammensetzung, die eine Verbindung nach einem der Ansprüche 1 bis 9 und ein pharmazeutisch unbedenkliches Verdünnungsmittel oder einen pharmazeutisch unbedenklichen Träger umfaßt.

12. Verbindung der allgemeinen Formel (I) 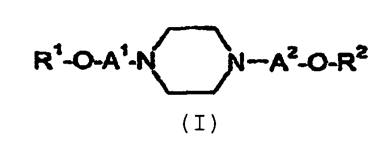 worin:
R¹ für eine Phenylgruppe steht, die durch ein oder zwei Fluoratome substituiert ist;
A¹ für (CH₂)ₘ oder (CH₂)ₘ₋₁C(=O) steht;
m für 2, 3, 4 oder 5 steht;
A² für (CH₂) oder C(=O) (CH₂) ₙ₋₁ steht;
n für 2, 3, 4 oder 5 steht und
R² für eine Phenylgruppe steht, die durch einen, zwei oder drei unabhängig voneinander unter C₁₋₂-Alkylendioxy, einer Hydroxylgruppe, einer Cyanogruppe, einer C₃₋₆-Cycloalkylgruppe, einer Halogen-C₁₋₉-alkylgruppe, einer C₁₋₄-Alkoxygruppe, einer C₁₋₄-Alkoxy-C₁₋₄-alkoxygruppe, einer Hydroxy-C₁₋₄-alkoxygruppe, einer C₁₋₄-Alkylsulfanylgruppe, einer C₁₋₄-Alkylsulfonylgruppe, einer Halogen-C₁₋₄-alkoxygruppe und einer Halogen-C₁₋₄-alkylsulfonylgruppe ausgewählte Substituenten substituiert ist;
oder ein pharmazeutisch unbedenkliches Salz davon zur Verwendung bei einem Verfahren der Behandlung einer neurologischen Störung oder einer psychiatrischen Störung, die mit Sigma-Rezeptoren in Verbindung gebracht worden ist, bei einem Patienten.

13. Verbindung nach Anspruch 12, wobei die neurologische Störung unter Gehirndefiziten nach Herz-Bypass-Chirurgie und Transplantation, Hirnischämie; Rückenmarksverletzung; Kopfverletzung; multipler Sklerose, Alzheimer-Krankheit; Chorea Huntington; amyotropher Lateralsklerose; AIDSinduzierter Demenz; Muskelspasmen; Konvulsionen; Drogentoleranz, -entzug und -einstellung; Augenschäden und Retinopathie; kognitiven Störungen; idiopathischer und drogeninduzierter Parkinson-Krankheit; Schmerzen und Bewegungsstörungen wie tardiver Dyskinesie ausgewählt ist und die psychiatrische Störung unter Schizophrenie, Angst und verwandten Störungen, Depression, manischdepressiven Krankheiten, Psychose und Zwangsstörungen ausgewählt ist.

14. Verbindung nach einem der Ansprüche 1 bis 9 zur Verwendung bei der Therapie.

15. Verwendung einer Verbindung der allgemeinen Formel (I) 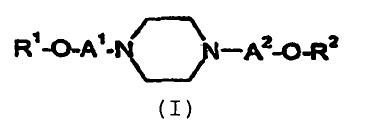 worin:
R¹ für eine Phenylgruppe steht, die durch ein oder zwei Fluoratome substituiert ist;
A¹ für (CH₂)ₘ oder (CH₂)ₘ₋₁C(=O) steht;
m für 2, 3, 4 oder 5 steht;
A² für (CH₂)ₙ oder C(=O)(CH₂)ₙ₋₁ steht;
n für 2, 3, 4 oder 5 steht und
R² für eine Phenylgruppe steht, die durch einen,
zwei oder drei unabhängig voneinander unter C₁₋₂-Alkylendioxy, einer Hydroxylgruppe, einer Cyanogruppe, einer C₃₋₆-Cycloalkylgruppe, einer Halogen-C₁₋₄-alkylgruppe, einer C₁₋₄-Alkoxygruppe, einer C₁₋₄-Alkoxy-C₁₋₄-alkoxygruppe, einer Hydroxy-C₁₋₄-alkoxygruppe, einer C₁₋₉-Alkylsulfanylgruppe, einer C₁₋₄-Alkylsulfonylgruppe, einer Halogen-C₁₋₄-alkoxygruppe und einer Halogen-C₁₋₄-alkylsulfonylgruppe ausgewählte Substituenten substituiert ist;
oder eines pharmazeutisch unbedenklichen Salzes davon bei der Herstellung eines Arzneimittels zur Behandlung einer neurologischen Störung oder einer psychiatrischen Störung, die mit Sigma-Rezeptoren in Verbindung gebracht worden ist.

16. Verwendung nach Anspruch 15, wobei die neurologische Störung unter Gehirndefiziten nach Herz-Bypass-Chirurgie und Transplantation, Hirnischämie; Rückenmarksverletzung; Kopfverletzung; multipler Sklerose, Alzheimer-Krankheit; Chorea Huntington; amyotropher Lateralsklerose; AIDSinduzierter Demenz; Muskelspasmen; Konvulsionen; Drogentoleranz, -entzug und -einstellung; Augenschäden und Retinopathie; kognitiven Störungen; idiopathischer und drogeninduzierter Parkinson-Krankheit; Schmerzen und Bewegungsstörungen wie tardiver Dyskinesie ausgewählt ist und die psychiatrische Störung unter Schizophrenie, Angst und verwandten Störungen, Depression, manischdepressiven Krankheiten, Psychose und Zwangsstörungen ausgewählt ist.

## Revendications

1. Composé de formule générale (I) 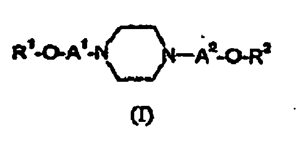 dans laquelle :
R¹ représente un groupement phényle qui est substitué par un ou deux atomes de fluor ;
A¹ représente (CH₂)ₘ ou (CH₂)ₘ-₁C (=O) ;
m est 2, 3, 4 ou 5 ;
A² représente (CH₂)ₙ ou C(=O) (CH₂)ₙ₋₁ ;
n est 2, 3, 4 ou 5 ; et
R² représente un groupement phényle qui est substitué par un, deux ou trois substituants choisis indépendamment parmi (1-2C)alkylènedioxy, un groupement hydroxyle, un groupement cyano, un groupement (3-6C)cycloalkyle, un groupement halogéno(1-4C)alkyle, un groupement (1-4C)alcoxy, un groupement (1-4C)alcoxy(1-4C)alcoxy, un groupement hydroxy(1-4C)alcoxy, un groupement (1-4C)alkylsulfanyle, un groupement (1-4C)alkylsulfonyle, un groupement halogéno(1-4C)alcoxy et un groupement halogéno(1-4C)alkylsulfonyle ;
ou sel pharmaceutiquement acceptable de celui-ci.

2. Composé selon la revendication 1, **caractérisé en ce que** R¹ représente un groupement 2-fluorophényle, 3-fluorophényle, 4-fluorophényle ou 2,4-difluorophényle.

3. Composé selon la revendication 1 ou la revendication 2, **caractérisé en ce que** m est 2 ou 3.

4. Composé selon la revendication 3, **caractérisé en ce que** m est 2.

5. Composé selon l'une quelconque des revendications 1 à 4, **caractérisé en ce que** A¹ représente (CH₂)ₘ.

6. Composé selon l'une quelconque des revendications 1 à 5, **caractérisé en ce que** n est 2 ou 3.

7. Composé selon l'une quelconque des revendications 1 à 6, **caractérisé en ce que** A² représente (CH₂)ₙ.

8. Composé selon l'une quelconque des revendications 1 à 7, **caractérisé en ce que** R² représente un groupement phényle qui est substitué par un ou deux substituants choisis indépendamment parmi un groupement cyano, un groupement fluoro(1-4C)alkyle, un groupement (1-4C)alkylsulfonyle, un groupement fluoro(1-4C)alcoxy ou un groupement fluoro(1-4C)alkylsulfonyle.

9. Composé selon la revendication 8, **caractérisé en ce que** R² représente 3-cyanophényle, 4-cyanophényle, 3-trifluorométhylphényle, 4-trifluorométhylphényle, 3-méthylsulfonylphényle, 4-méthylsulfonylphényle, 3-trifluorométhylsulfonylphényle ou 4-trifluorométhylsulfonylphényle.

10. Procédé de préparation d'un composé selon l'une quelconque des revendications 1 à 9, **caractérisé en ce qu'**il comprend
a) la réaction d'un composé de formule générale (II) 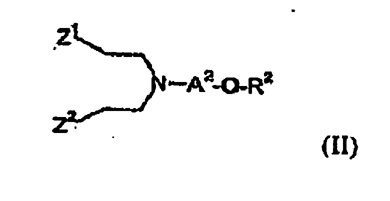 dans laquelle chacun de Z¹ et Z² représente indépendamment un atome ou groupement partant, ou un composé correspondant dans lequel un ou plusieurs substituants sur R² sont protégés, avec un composé de formule générale (III)
R¹-O-A¹-NH₂ X (III)
b) la réaction d'un composé de formule générale (IV) 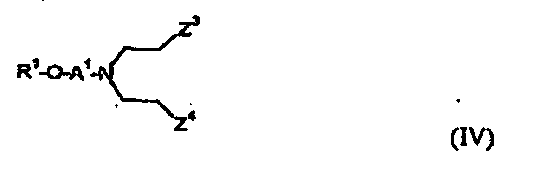 dans laquelle chacun de Z³ et Z⁴ représente indépendamment un atome ou groupement partant, avec un composé de formule générale (V)
H₂N-A²-O-R^{Z} (V)
ou un composé correspondant dans lequel un ou plusieurs substituants sur R² sont protégés ;
c) la réaction d'un composé de formule générale (VI) 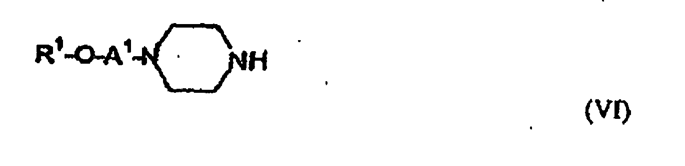 avec un composé de formule générale (VII)
Z⁵-A²-O-R² (VII)
dans laquelle Z⁵ représente un atome ou groupement partant ;
d) la réaction d'un composé de formule (VIII) 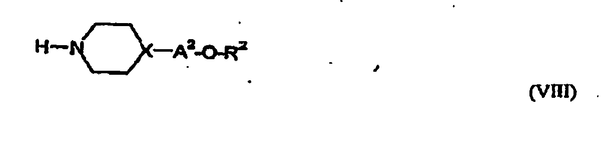 avec un composé de formule générale (IX)
R¹-O-A¹-Z⁶ (IX)
dans laquelle Z⁶ représente un atome ou groupement partant ; ou
e) pour un composé de formule (I) dans laquelle A¹ représente (CH₂) ₘ et A² représente (CH²) ₙ, la réduction d'un composé correspondant de formule (I) dans laquelle A¹ représente (CH₂)ₘ₋₁C (=O) et/ou A² représente C(=O) (CH₂)ₙ₋₁ ;
suivies de l'élimination de tout groupement protecteur et, éventuellement, la formation d'un sel pharmaceutiquement acceptable.

11. Composition pharmaceutique, **caractérisée en ce qu'**elle comprend un composé selon l'une quelconque des revendications 1 à 9, et un diluant ou un support pharmaceutiquement acceptable.

12. Composé de formule générale (I) 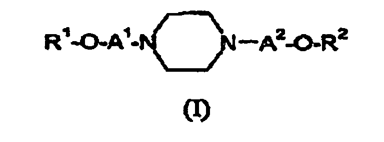 dans laquelle :
R¹ représente un groupement phényle qui est substitué par un ou deux atomes de fluor ;
A¹ représente (CH₂)ₘ ou (CH₂)ₘ₋₁C(=O) ;
m est 2, 3, 4 ou 5 ;
A² représente (CH₂)ₙ ou C(=O) (CH₂)ₙ₋₁ ;
n est 2, 3, 4 ou 5 ; et
R² représente un groupement phényle qui est substitué par un, deux ou trois substituants choisis indépendamment parmi (1-2C)alkylènedioxy, un groupement hydroxyle, un groupement cyano, un groupement (3-6C)cycloalkyle, un groupement halogéno(1-4C)alkyle, un groupement (1-4C)alcoxy, un groupement (1-4C)alcoxy(1-4C)alcoxy, un groupement hydroxy(1-4C)alcoxy, un groupement (1-4C)alkylsulfanyle, un groupement (1-4C)alkylsulfonyle, un groupement halogéno(1-4C)alcoxy et un groupement halogéno(1-4C)alkylsulfonyle ;
ou sel pharmaceutiquement acceptable de celui-ci,
destiné à être utilisé dans une méthode de traitement d'un trouble neurologique ou d'un trouble psychiatrique qui a été lié aux récepteurs sigma chez un patient.

13. Composé selon la revendication 12, **caractérisé en ce que** le trouble neurologique est choisi parmi les déficits cérébraux suivant une chirurgie et une greffe par pontage cardiaque ; l'ischémie cérébrale ; le traumatisme médullaire ; le traumatisme crânien ; la sclérose en plaques ; la maladie d'Alzheimer ; la chorée de Huntington ; la sclérose latérale amyotrophique ; la démence induite par le SIDA ; les spasmes musculaires ; les convulsions ; la tolérance, le manque, et le sevrage de drogues ; la lésion oculaire et la rétinopathie ; les troubles cognitifs ; la maladie de Parkinson idiopathique et induite par un médicament ; la douleur ; et les troubles du mouvement tels que la dyskinésie tardive, et le trouble psychiatrique est choisi parmi la schizophrénie, l'anxiété et les troubles apparentés, la dépression, les troubles bipolaires, la psychose, et les troubles obsessifs compulsifs.

14. Composé selon l'une quelconque des revendications 1 à 9, destiné à être utilisé en thérapie.

15. Utilisation d'un composé de formule générale (I) 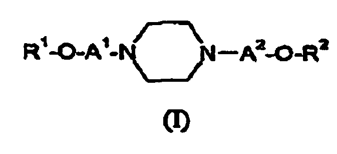 dans laquelle :
R¹ représente un groupement phényle qui est substitué par un ou deux atomes de fluor ;
A¹ représente (CH₂)ₘ ou (CH₂) ₘ₋₁C (=O) ;
m est 2, 3, 4 ou 5 ;
A² représente (C_{H2})ₙ ou C (=O) (CH₂) ₙ₋₁ ;
n est 2, 3, 4 ou 5 ; et
R² représente un groupement phényle qui est substitué par un, deux ou trois substituants choisis indépendamment parmi (1-2C)alkylènedioxy, un groupement hydroxyle, un groupement cyano, un groupement (3-6C)cycloalkyle, un groupement halogéno(1-4C)alkyle, un groupement (1-4C)alcoxy, un groupement (1-4C)alcoxy(1-4C)alcoxy, un groupement hydroxy(1-4C)alcoxy, un groupement (1-4C)alkylsulfanyle, un groupement (1-4C)alkylsulfonyle, un groupement halogéno(1-4C)alcoxy et un groupement halogéno(1-4C)alkylsulfonyle ;
ou d'un sel pharmaceutiquement acceptable de celui-ci, dans la fabrication d'un médicament destiné au traitement d'un trouble neurologique ou d'un trouble psychiatrique qui a été lié aux récepteurs sigma.

16. Utilisation selon la revendication 15, **caractérisée en ce que** le trouble neurologique est choisi parmi les déficits cérébraux suivant une chirurgie et une greffe par pontage cardiaque ; l'ischémie cérébrale ; le traumatisme médullaire ; le traumatisme crânien ; la sclérose en plaques ; la maladie d'Alzheimer ; la chorée de Huntington ; la sclérose latérale amyotrophique ; la démence induite par le SIDA ; les spasmes musculaires ; les convulsions ; la tolérance, le manque, et le sevrage de drogues ; la lésion oculaire et la rétinopathie ; les troubles cognitifs ; la maladie de Parkinson idiopathique et induite par un médicament ; la douleur ; et les troubles du mouvement tels que la dyskinésie tardive, et le trouble psychiatrique est choisi parmi la schizophrénie, l'anxiété et les troubles apparentés, la dépression, les troubles bipolaires, la psychose, et les troubles obsessifs compulsifs.
